Europäisches Patentamt

European Patent Office

Office européen des brevets

(11) Publication number: **0 322 768**
**A1**

## (12) EUROPEAN PATENT APPLICATION

(21) Application number: 88121515.6

(51) Int. Cl.⁴: **C12P 21/00**

(22) Date of filing: 22.12.88

(30) Priority: 24.12.87 JP 327877/87

(43) Date of publication of application:
05.07.89 Bulletin 89/27

(84) Designated Contracting States:
**DE IT NL**

(71) Applicant: **NIPPON SHINYAKU COMPANY, LIMITED**
**14, Kisshoin Nishinosho Monguchicho Minami-ku Kyoto-shi Kyoto 601(JP)**

(72) Inventor: **Yano, Junichi**
**2-840-86 Sahodai**
**Nara 630(JP)**
Inventor: **Kawakatsu, Hisaaki**
**1-5-2-404 Okaya**
**Otsu 520-21(JP)**

(74) Representative: **Wey, Hans-Heinrich, Dipl.-Ing.**
**Patentanwälte Wey & Partner**
**Widenmayerstrasse 49**
**D-8000 München 22(DE)**

(54) Monoclonal antibody and a method for production thereof as well as a method for application thereof.

(57) A monoclonal antibody being capable of specifically recognizing a protein belonging to a 36K family can be used in a method for identifying of quantitatively determining a protein belonging to a 36K family; such a monoclonal antibody can be produced by chemically synthesizing a peptide corresponding to an amino acid sequence at the N-end region of a protein belonging to the 36K family and collecting an antibody using as an antigen said peptide which may be optionally bound to a carriert.

EP 0 322 768 A1

## MONOCLONAL ANTIBODY AND A METHOD FOR PRODUCTION THEREOF AS WELL AS A METHOD FOR APPLICATION THEREOF

The present invention relates to a novel monoclonal antibody and more particularly, to a monoclonal antibody having excellent utility as drugs by recognizing a specific site of a membrane cytoskeleton protein which is present around cell membrane and considered to take part in the information transfer system.

In recent years, it has been made clear that the information transfer system and the cytoskeleton system which were hitherto considered to take quite different roles were closely related to from each other, due to remarkable development of molecular biological technique.

It has been clarified by many studies that inter alia, a series of proteins called membrane cytoskeleton protein function as a first carrier for transferring the information into the cells. after physiological stimulation from external source outside cells is converted into a definite chemical information by a receptor on the cell surface.

Among the aforesaid membrane cytoskeleton protein, a series of proteins having a molecular weight of approximately 36,000 are called a 36K protein group (which is referred to as "36K family" in the specification) and are known to be peculiar proteins that exhibit multiple functions to take part in transfer of information such as transformation, inflammation, immunity, etc.

The proteins collectively referred to as the 36K family are considered to cause interaction associated with lipid and calcium, together with proteins such as actin, fodrin, etc. present therearound.

Various investigations were made on the 36K family; presence of Calpactin 1 (also called lipocortin 2), Calpactin 2 (also called lipocortin 1), etc. and their primary structures have been made clear [Cell, 46, 201-212 (1986), Cell, 46, 191-199 (1986)].

In addition, with respect to protein called IBC (inhibitor of blood coagulation), its primary structure has been clarified [J. Biochem. (Tokyo), 102, 1261-1273 (1987(].

Besides, with respect to other proteins belonging to the 36K family, their presence is suggested and it is expected to clarify cytophysiological role of the 36K family in function and participation in various diseases.

Relationship between the 36K family and transformation was already reported [Proc. Natl. Acad. Sci., 76, 5212-5216 (1979)], from which it has been made clear that in culture cells transformed by carcinogenic gene-bearing virus, introduction of phosphorylation into their tyrosine residues markedly increase. It is believed that Calpactin 1 described above was observed in this study but it is yet unclear as to how the increased phosphorylation in transformation incudes infinite proliferation of cells, etc.

Relationship between physiological significance of inflammation and the 36K family is also studied.

Prostaglandin, leukotriene, PAF, etc. which are chemical substances for transferrring inflammation in vivo are released from cell membrane by phospholipase $A_2$ in inflammatory site. It has been made clear that antiinflammatory proteins heretofore termed lipomodulin, macrocortin, renocortin, etc. are members of the 36K family and it has been confirmed that their antiinflammatory activity is exhibited by inhibiting the aforesaid phospholipase $A_2$.

This phospholipase $A_2$ inhibition activity is a property possessed by all of hitherto known proteins belonging to the 36K family. It is also known that this activity is lost by phosphorylation.

Relationship between physiological mechanism of immunity and the 36K family is also studied.

It has been made clear that glycosylation inhibiting factor (GIF) produced by suppressor T cell, macrophage, etc. is a member of the 36K family [Proc. Natl. Acad. Sci., 83, 160-164 (1986)], which leads to future subject to be studied on relation to immune diseases such as allergy, rheumatoid, etc.

It is also considered that intracellular proteins called calelectrin, chromobindin,, calcimedin, endonexin, annexin, etc. would belong to the 36K family and it is assumed that they would take part in transfer of physiologically various information.

As stated above, a series of cellular proteins collectively referred to as the 36K family are substances which have great significance from a physiological viewpoint. To identify and quantitatively determine them was an extremely important problem to be investigated which would directly relate to development of excellent drugs.

(Prior Art)

As described above, it is extremely important for development of drugs to establish a means for identifying and quantitatively determining proteins belonging to the 36K family.

As means for identifying and quantitatively determining a series of proteins having similar structures in which independent functions are expected, the following can be considered so far.

(1) A method for indirectly identifying the protein by recognizing its DNA sequence using DNA probe;

(2) A method for recognizing the protein by a so-called antigen-antibody reaction through harvesting a monoclonal antibody (hereafter sometimes referred to as "Mb");

The method utilizing Mb is considered to be an ideal analytical means since Mb is capable of directly binding with an expressed protein and each can recognize the only one antigen determinant.

Among the 36K family, Mb to human Calpactin 1 was known [Mol. Cell. Biol., 6, 2745-2751 (1986)]. However, according to this method, immunization was effected using crude protein so that it was unclear what site of the protein Mb recognized and therefore, the crude protein was unsuitable as an antibody for examining a specific function.

Further, Mb to rabbit lipomodulin was also known [ J. Immunol., 132, 1286-1293 (1984)]. However, its recognition site in the protein was likewise unclear as described above and similar problem was involved.

(Problems to be solved by the Invention)

Survey of gene sequences of Calpactin 1 and Calpactin 2 reveals that amino acid sequences of these proteins contain a repeating structure of 4 domains similar to each other and on the definite domains from the N-end thereof (end having an amino group) (referred to as "N-end domain" in the specification) are differnet from each other.

Tyrosine-specific phosphorylase, a site for phosphorylation with C-kinase, a site in which lipid modification is expected, a site for being hydrolyzed by protease, etc. are concentrated upon the N-terminal domain.

From the foregoing, it could be easily assumed that among a series of the proteins belonging to the 36K family, the N-end domain would be in charge of the function characteristic to each protein.

Thus, if Mb capable of specifically recognizing the N-end domain can be obtained, (1) a mechanism of the protein belonging to the 36K family on disease could be clarified and (2) identification could be made by-denying possibilities of cross reaction between proteins belonging to the 36K family.

Under these circumstances, the present inventors have made extensive investigations to obtain Mb capable of specifically recognizing the N-terminal domain.described above and have finally reached the present invention.

(Means for solving the Problems)

In the present invention, the optimum peptide is chemically synthesized, in view of antigenicity at the functional site out of the N-terminal domain.

For this reason, antigenicity is expected by means of a computer analysis through homology survey, etc. using amino acid sequence expected from DNA sequence of the 36K family and various peptides are chemically synthesized.

The peptide used for immunization herein can also be purified from the 36K family protein purified, in case that a cleavage site with an appropriate -protease decomposition enzyme is present.

In the present invention, the thus acquired peptide can be immunized by binding to an appropriate carrier, for example, keyhole limpet hemocyanin , etc.

Further, depending upon purpose, a gene sequence of the N-end domain of the 36K family protein is bound to E. coli plasmid at, for example, a $\beta$-galactosidase portion to express the same as a fused protein; the fused protein can be used after purification.

For the binding of peptide to a carrier, an appropriate chemical reagent, for example, glutaraldehyde, carbodiimide, MBS (m-Maleimidobenzoyl-N-hydroxysuccinimide ester) , etc. can be used.

Hereafter, a method for obtaining Mb in accordance with the present invention is described using the thus obtained protein as an antigen.

Firstly, preparation of immunized cells is explained.

The immunized cells can be obtained by administering the antigen in the animal body and collecting cells from the animal.

As the animal, animals such as mice or the like hitherto ordinarily provided for experiments can be used. It is desired to administer the antigen intraperitoneally, etc. It is appropriate to administer as an admixture with Freund's complete adjuvant in a conventional manner but in the present invention, it is preferred that administration be repeated several times with intervals of several weeks after the administration. It may be sufficient that a time period for the administration be 2 weeks and the number of administration is preferably 2 to 4 times.

Then, the animal is sacrificed and organs, for example, spleen, etc. are withdrawn to give cells in a conventional manner.

Next, cell fusion to myeloma cells for purpose of imparting a proliferation function to the immunized cells is described.

The myeloma cells used herein can be obtained by culturing, for example, SP2/0-Ag14 strain, etc. in a medium containing, for example, FCS (bovine feral serum). Preferably, cells at the exponential growth phase are used.

For cell fusion, the immunized cells are mixed with myeloma cells in a cell count ratio ranging from 1 : 1 to 10 : 1 followed by fusion using a fusing agent such as polyethylene glycol, etc. or electric stimulation, etc.

The cells after fusion are cultured in so called HAT medium supplemented with hypoxanthine, aminopterin and thymidine immediately or after preincubation in ordinary medium, whereby only the cells fused in combination of immunized cells and myelomal cells can be selected.

In the present invention, antibody-producing cells can be selected while confirming an antibody titer by, for example, enzyme-linked immunoassay (ELISA), etc.

The cells in which the antibody titer could be confirmed are repeatedly cultured in a well plate or Petri dish in a conventional manner such the limiting dilution method, the soft agar method, etc., during which antibody productivity, immunochemical test of produced antibody, antigen specificity test and the like are performed thereby to effect selection. Besides, selection can also be made using a micro manupilator or a cell sorter.

The cells in accordance with the present invention can continuously produce Mb in accordance with the present invention. Accordingly, in the case of utilizing Mb in accordance with the present invention, the supernatant of the culture solution in accordance with the present invention can be utilized as the Mb solution in accordance with the present invention directly as it is.

Further, Mb in accordance with the present invention can also be produced by administering antibody-producing cells in animal body (for example, intraperitoneally) such as mice, etc. usually provided for test and collecting body fluid of the animal, for example, ascites, etc.

To purify and collect Mb in accordance with the present invention from the culture solution of the cells in accordance with the present invention or ascites, Mb can be harvested by means of, for example, precipitation with ammonium sulfate, ion exchange chromatography, etc.

The thus harvested Mb can be stored as a stable substance by adding, for example, various buffer solutions and if necessary, salt and azide, etc., or by freeze drying, etc.

Identification of each class of immunoglobulin of these antibodies can be performed by ELISA using anti-class-specific antibody.

With respect to the thus obtained Mb capable of each N-terminal domain of the 36K family, it can be confirmed that Mb specifically binds to each protein belonging to the 36K family by the following immunobiochemical means.

A technique for identification using Mb in accordance with the present invention by applying western blotting analysis thereto is explained below.

This technique can be applied to identification and quantitative determination of peptide in living organism-derived specimen. That is, a living organism-derived specimen is fractionated by SDS polyacrylamide gel electrophoresis in a conventional manner followed by blotting on a nylon membrane or a nitrocellulose membrane. The peptide transferred onto the membrane is bound to Mb, which is then bound to protein A labeled with $^{35}$S or $^{125}$I. In this case, an excess of Mb and protein A are removed every time by thorough washing.

By doing this, $^{35}$S or $^{125}$I is theoretically present in proportion to the amount of Mb at the position of the peptide, correspondingly to specificity of Mb used. $^{35}$S or $^{125}$I on the membrane is detected by an RI scanner or an autoradiography, whereby identification and quantitative determination of the peptide can be performed.

Next, a technique for identification of the peptide characterized by applying a means of RIP (radioimmunoprecipitation) is described below.

In RIP, a cell specimen is cultured in a culture solution containing methionine lablled with, for example, $^{35}$S. etc. thereby to incorporate the label into the cell specimen. Then, a buffer solution for solubilization is added thereto and lysate is collected. Mb in accordance with the present invention is added to the lysate.

By mixing, an antigen-antibody reaction is completed.

Thereafter, protein A-Sepharose, etc. is added in a conventional manner. By repeating centrifugation and washing with buffer, impurities other than the peptide in accordance with the present invention are removed. Then, electrophoresis is performed in a conventional manner to detect the radioisotope. In case that the peptide is present in the specimen cells, bands corresponding to specificity of Mb used appear.

Next, a technique for detecting the peptide in living organism characterized by applying immunocytochemistry is explained below.

In the case of culture cells, the cells are immobilized with an appropriate fixing solution such as a formaldehyde-phosphate buffered saline (PBS) solution, etc., whereby a Triton treatment is effected to provide as samples. Mb in accordance with the present invention is added to these samples to complete an antigen-antibody reaction followed by thoroughly washing with phosphate buffer. After biotin-bound antiimmunoglobulin is reacted as a secondary antibody, through washing follows. Fluorescence-labelled avidin is reacted with the complex of peptide-Mb-biotin-bound secondary antibody formed. Its fluorescence is observed by a fluorescence microscope to detect the peptide in the cells.

In the case of pathological tissue, the tissue is fixed with formalin or other appropriate fixing solution to make a slice or a freeze dried slice is then fixed with formaldehyde, etc., if necessary, further treated with a surface active agent; the thus obtained specimen is provided as a sample. The sample is treated in a similar manner to conduct immunocytochemistry.

According to the present invention, it is possible to obtain Mb capable of specifically recognizing the N-end domain of each protein in the 36K family. By applying immunobiochemical techniques such as the western blotting, immunocytochemistry, etc. described above in detail thereto, each protein belonging to the 36K family can be identified even though it is present in a trace amount. This means that an epoch-making method has been acquired to trace function of each protein in charge, a ratio of presence in each tissue, state of distribution (processing, phosphorylation), etc.

Further this Mb is capable of specifically recognizing the N-end domain of each protein in the 36K family and it is thus possible to incorporate the system of inhibiting phosphorylation, sugar, modification of lipid, binding to 10K protein in Calpactin 1, etc. coupled with a suitable antibody. Therefore, these phenomena can be useful materials to study as to how the phenomena take part in expressing the function of each protein of the 36K family.

From the foregoing matters, Mb of the present invention is useful for clarification of information transfer mechanism in vivo in diseases such as transformation, immune diseases, inflammation, etc. and is considered to be essential for establishing therapy of these diseases.

(Examples)

Hereafter, the present invention will be described in more detail with reference to the examples below but these examples are simply to illustrate embodiments of the present invention and the present invention is not deemed to be limited thereto.

Example 1

Preparation of Mb to N-end domain of Calpactin 1

(1) Production of antigen

Among amino acid sequence expected from gene sequence, 28 amino acids from the first methionine to the 28th lysine were chemically synthesized in a conventional manner.

A solution of 10 mg of this peptide in 2 ml of purified distilled water was mixed with 4 mg/ml of BSA solution (Fraction V manufactured by Sigma Inc. was used) and water soluble carbodiimide (manufactured by Protein Study Promortion Association) as a coupling agent in a final concentration of 0.2 M was added to the mixture.

While maintaining pH at 6.0, coupling reaction was performed for 30 minutes to an hour until change in pH was not observed. Then, dialysis was performed at 4°C with PBS (-) and unreacted matters were removed through a gel filtration column. Thus, 12 mg of peptide-BSA complex was obtained.

The amino acid sequence (28 amino acids from the first to 28th) corresponding to the N-terminal domain of Calpactin 1 was known and is as follows.

Met-Ser-Thr-Val-His-Glu-Ile-Leu-Cys-Lys-Leu-Ser-Leu-Glu-Gly-Asp-His-Ser-Thr-Pro-Pro-Ser-Ala-Tyr-Gly-Ser-Val-Lys

## (2) Production of immunized cells

A mixture of 0.1 ml of a solution containing 100 µg of the substance obtained in (1) and 0.1 ml of Freund's complete adjuvant (manufactured by DIFCO Co., Ltd.) was intraperitoneally administered to BALB/c female mouse of 10 weeks age. Thereafter, a solution mixture of the solution described above and incomplete adjuvant (manufactured by DIFCO Co., Ltd.) was intraperitoneally administered twice in a 2 week interval. Then, the mouse was killed by servical dislocation and the spleen was aseptically withdrawn.

On a selector (manufactured by BELCO Co., Ltd.), about 1 g of the spleen described above was put and passed through a mesh of about 10 µ, while supplying Dulbecco modified MEM (D-MEM) medium, to give spleen cells in a state suspended in D-MEM. The suspension was centrifuged at 100G for 5 minutes to collect the spleen cells.

The cells described above were added to 1 ml of a solution obtained by adding 20 mM HEPES buffer (pH 7.4) to 0.84% of ammonium chloride solution to cause hemolysis. The cells were obtained by centrifugation at 1,000G for 5 minutes. The cells were resuspended in D-MEM.

## (3) Production of myleoma cells

8-Azaguanine-resistant and immunoglobulin non-secretion type mouse myeloma cell line SP2/0-Ag14 strain was cultured in D-MEM medium containing 20% bovine fetal serum (FCS) in an incubator at 37°C under 10% $CO_2$. Cells in the exponential growth phase were collected and used for the following operation.

Only the cells were collected by centrifugation at 1,000G for 5 minutes and further suspended in D-MEM medium. The cell count was counted with a hemacytometer. After centrifugation again at 1,000G for 5 minutes, the cells were resuspended in D-MEM medium.

## (4) Cell fusion

The D-MEM medium containing $10^8$ to $3 \times 10^8$ obtained in (1) was mixed with the D-MEM medium containing $10^8$ of myeloma cells obtianed in (3). After homogenizing, the mixture was centrifuged at 1,000G for 5 minutes. The supernatant was removed and the precipitates were obtained and 1 ml of a solution containing 25% (w/v) polyethylene glycol 1500 (PEG 1500, manufactured by Boehringer Co., Ltd.) and 37.5 mM of HEPES buffer was dropwise added to the precipitates over a minute. Then, the mixture was gradually diluted with D-MEM medium to make the whole 10 ml.

Thereto was added 10 ml of D-MEM medium containing 20% FCS followed by centrifugation at 1,000G for 5 minutes. 20% FCS-containing D-MEM was added to the obtained cells in $10^6$ cells/ml. The mixture was dispensed in a 24 well culture plate manufactured by Corning Glass Inc. in a rate of 1 ml/well followed by culturing at 37°C for 24 hours under 10% $CO_2$ in an incubator.

Then, HAT solution was added to remove cells other than the fused cells. Incubation was continued for further 2 weeks.

## (5) Measurement of antibody titer by ELISA

Each well of microtiter plate (No. 001-010-2101 manufactured by Dinatec Laboratories Co., Ltd.) was coated with 1 µg/ml of peptide obtained in (1). The supernatant, 150 µl, containing the antibody as an analyte was taken in the well, allowed to stand at 37°C for 2 hours in the incubator and then washed with PBS (phosphate buffer-saline. Peroxidase-bound goat anti-mouse full antibody was added thereto and allowed to stand at 37°C for an hour in the incubator. After washing with PBS, a coloring agent (ABTS) was added to form a color for 15 minutes. A stopping solution (0.1 M citrate-0.02% sodium azide) was added to stop the reaction. Thereafter, absorbancy of the well was measured with a multiscanner manufactured by Titertec Co., Ltd. to determine the antibody titer.

## (6) Selection of antibody-producing cells

The cells in the well which were confirmed by ELISA was inoculated on soft agar medium in a Petri dish of 60 mmØ. Incubation was performed at 37°C under 10% $CO_2$ for 2 weeks in an incubator to form a colony.

The formed colony was taken and put on a 24 well culture plate. Antibody activity was again confirmed by ELISA. The cells in the well which was confirmed by the antibody activity was inoculated on soft agar medium in a Petri dish of 60 mmØ. Incubation was performed at 37°C under 10% $CO_2$ for 2 weeks in an incubator to form a colony. The formed colony was taken and put on a 24 well culture plate. Antibody activity was again confirmed by ELISA to select useful cells.

## (7) Collection of antibody

(1) The antibody-producing cells obtained in (6) are capable of always producing the antibody of the present invention. Accordingly, the supernatant of the culture solution in which the antibody-producing cells were cultured can be used directly as a solution of the antibody of the present invention.

(2) Ammonium sulfate was added to the culture solution of the antibody-producing cells obtained in (6) in a final concentration of 30%. After centrifugation, the precipitates were collected and 20 mM of phosphate buffer showing pH of 7.4 was added thereto. Dialysis was performed using the same phosphate buffer (containing 0.02% sodium azide) to remove ammonium sulfate. The solution after the dialysis was freeze dried to give white powders.

(3) Pristane 0.5 mg. was intraperitoneally injected to BALB/C male mouse and the mouse was bred for 2 weeks. The antibody-producing cells obtained in (6) were injected to the mouse in $10^6$ to $3 \times 10^6$ cells/mouse followed by breeding for 10 days. About 10 ml of the ascites retained in the peritoneal cavity was collected through a syringe.

Ammonium sulfate was added to the ascites in a final concentration of 30%. Then, 20 mM of phosphate buffer showing pH of 7.4 was added thereto. Dialysis was performed using the same phosphate buffer (containing 0.02% sodium azide) to remove ammonium sulfate. The solution after the dialysis was freeze dried to give white powders.

## (8) Identification of each class of immunoglobulin

Identification of each class of immunoglobulin was performed as follows. Class-specific antibodies (as IgA, IgGl, IgG2a, IgG2b, IgG3 and IgM were used those manufactured by Biorad Co., Ltd.) were added followed by reacting at 37°C for 2 hours. Then, the system was thoroughly washed with PBS. Thereto was added peroxidase-bound goat anti-mouse full antibody. The mixture was allowed to stand at 37°C for an hour in an incubator. After washing with PBS, a coloring agent (ABTS) was added to form a color for 15 minutes, whereby each class was identified. The results are in part shown in Table 1.

Table· 1

| Reference No. | Cell Line | Subclass |
|---|---|---|
| 1 | 3.10H | G2b |
| 2 | 3.11A | G2b |
| 3 | 4.5 E | G2a |
| 4 | 4.7 G | G2b |
| 5 | 4.9 H | G2b |
| 6 | 5.2 A | G2a |
| 7 | 5.2 C | G2b |
| 8 | 5.10E | G2b |
| 9 | 6.4 C | G2b |
| 10 | 6.7 C | G3 |
| 11 | 6.9 F | G1 |
| 12 | 9.2 E | G2b |
| 13 | 10.9 B | G3 |
| 14 | 10.11A | G2b |
| 15 | 11.2 F | G2b |

Example 2

Production of Mb to the N-end domain of Calpactin 2

The amino acid sequence (23 amino acids from the l3th to 35th) corresponding to the N-terminal domain of Calpactin 2 was known and is as follows.

Phe-Ile-Glu-Asn-Glu-Glu-Gln-Glu-Tyr-Val-Gln-Thr-Val-Lys-Ser-Ser-Lys-Gly-Gly-Pro-Gly-Ser-Ala

Peptide having the amino acid sequence composed of the 23 amino acids were obtained by chemical synthesis.

Subsequently, procedures were carried out in a manner similar to Example 1 to give the objective Mb.

Identification of each class of immunoglobulin was performed in a manner similar to Example 1 to give the results shown in Table 2.

Table 2

| Reference No. | Cell Line | Subclass |
|---|---|---|
| 1 | 1.5 A | G2b |
| 2 | 2.5 C | G1 |
| 3 | 2.10D | G2a |
| 4 | 5.3 B | G2a |
| 5 | 5.5 E | G3 |
| 6 | 5.8 A | G2b |
| 7 | 7.1 B | G1 |
| 8 | 7.5 D | G2a |
| 9 | 10.5 A | G2b |
| 10 | 10.10A | M |

Example 3

Production of Mb to the N-terminal domain of IBC ·

The amino acid sequence (22 amino acids from the first to 22th) corresponding to the N-end domain of IBC was known and is as follows.

Met-Ala-Gln-Val-Leu-Arg-Gly-Thr-Val-Thr-Asp-Phe-Pro-Gly-Phe-Asp-Glu-Arg-Ala-Asp-Ala-Glu

Peptide having the amino acid sequence composed of the 22 amino acids were obtained by chemical synthesis.

Subsequently, procedures were carried out in a manner similar to Example 1 to give the objective Mb.

Identification of each class of immunoglobulin was performed in a manner similar to Example 1 to give the results shown in Table 3.

Table 3

| Reference No. | Cell Line | Subclass |
|---|---|---|
| 1 | 1.4 B | G3 |
| 2 | 2.2 B | G2a |
| 3 | 2.50A | G2a |
| 4 | 5.2 B | G2b |
| 5 | 5.5 D | G2a |
| 6 | 5.7 A | G2a |
| 7 | 6.1 B | G2b |
| 8 | 7.8 D | G3 |
| 9 | 11.5 A | G2a |
| 10 | 12.30B | G2a |

**Claims**

1. A monoclonal antibody capable of specifically recognizing a protein belonging to a 36K family.

2. A method for producing a monoclonal antibody capable of specifically recognizing a protein belonging to a 36K family which comprises chemically synthesizing a peptide corresponding to an amino acid sequence at the N-end region of a protein belonging to the 36K family and collecting an antibody using as an antigen said peptide which may be optionally bound to a carrier.

3. A method for identifying or quantitatively determining a protein belonging to a 36K family using a monoclonal antibody capable of specifically recognizing a protein belonging to the 36K family.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | THE JOURNAL OF CELL BIOLOGY, vol. 105, no. 5, November 1987, pages 2111-2121, The Rockefeller University Pres.; L. ZOKAS et al.: "The calpactin light chain is tightly linked to the cytoskeletal form of calpactin I: studies using monoclonal antibodies to calpactin subunits" * Pages 2111-2113 * | 1,3 | C 12 P 21/00 |
| Y | Idem | 2 | |
| Y | JOURNAL OF IMMUNOLOGY, vol. 138, no. 10, 15th May 1987, pages 3332-3337, The American Association of Immunologists, US; P. MOTTE et al.: "Monoclonal antibodies distinguish synthetic peptides that differ in one chemical group" * Abstract * | 2 | |
| X,D | MOLECULAR AND CELLULAR BIOLOGY, vol. 6, no. 7, July 1986, pages 2745-2751, The American Society of Microbiology, US; C.M. ISACKE et al.: "Modulation of p36 phosphorylation in human cells: studies using anti-p36 monoclonal antibodies" * Page 2745 * ---                                   -/- | 1,3 | **TECHNICAL FIELDS SEARCHED (Int. Cl.4)**  C 12 P A 61 K |

The present search report has been drawn up for all claims

| Place of search | Date of completion of the search | Examiner |
|---|---|---|
| THE HAGUE | 05-04-1989 | ALVAREZ Y ALVAREZ C. |

EPO FORM 1503 03.82 (P0401)

**European Patent Office**    EUROPEAN SEARCH REPORT    Application Number

EP  88 12 1515

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | JOURNAL OF IMMUNOLOGY, vol. 132, no. 3, March 1984, pages 1286-1293, The American Association of Immunologists, US; M. IWATA et al.: "Modulation of the biologic activities of IgE-binding factor. V. The role of glycosylation-enhancing factor and glycosylation-inhibiting factor in determining the nature of IgE-binding factors" * Page 1288 * | 1 | |
| X,D | JOURNAL OF BIOCHEMISTRY, vol. 102, no. 5, 1987, pages 1261-1273; A. IWASAKI et al.: "Structure and expression of cDNA for and inhibitor of blood coagulation isolated from human placenta: a new lipocortin-like protein" * Abstract; pages 1263-1264; Results * | 1 | |
| X | JOURNAL OF NEUROCHEMISTRY, vol. 48, suppl., 1987, abstract C; W. FIEDLER et al.: "Calelectrin, a calcium-binding protein: immunohistochemical localization using a new monoclonal antibody" * Whole abstract * | 1,3 | **TECHNICAL FIELDS SEARCHED (Int. Cl.4)** |
| A | CELL, vol. 46, 18th July 1986, pages 191-199, Cell Press; K.-S. HUANG et al.: "Two human 35 kd inhibitors of phospholipase A2 are related to substrates of pp60v-src and of the epidermal growth factor receptor/kinase" -/- | 1 | |

The present search report has been drawn up for all claims

| Place of search | Date of completion of the search | Examiner |
|---|---|---|
| THE HAGUE | 05-04-1989 | ALVAREZ Y ALVAREZ C. |

European Patent
Office

**EUROPEAN SEARCH REPORT**

## DOCUMENTS CONSIDERED TO BE RELEVANT

| | Category | Citation of document with indication, where appropriate, of relevant passages | Relevant to claim | CLASSIFICATION OF THE APPLICATION (Int. Cl.4) |
|---|---|---|---|---|
| | A | CELL, vol. 46, 18th July 1986, pages 201-212, Cell Press; C.J.M. SARIS et al.: "The cDNA sequence for the protein-tyrosine kinase substrate p36 (calpactin I heavy chain) reveals a multidomain protein with internal repeats"<br><br>--- | | |
| | X,P | BIOCHEMISTRY, vol. 27, no. 6, 1988, pages 2069-2076, American Chemical Society; J. GLENNEY et al.: "Antibodies to the N-terminus of calpactin II (p35) affect Ca2+ binding and phosphorylation by the epidermal growth factor receptor in vitro"<br>* Whole article *<br><br>----- | 1,3 | |
| | | | | **TECHNICAL FIELDS SEARCHED (Int. Cl.4)** |

**The present search report has been drawn up for all claims**

| Place of search | Date of completion of the search | Examiner |
|---|---|---|
| THE HAGUE | 05-04-1989 | ALVAREZ Y ALVAREZ C. |

EPO FORM 1503 03.82 (P0401)